# FASCICULE DE BREVET EUROPEEN

(11) **EP 0 422 167 B1**
(45) Date de publication et mention de la délivrance du brevet: **30.06.1993**
(21) Numéro de dépôt: 90906105.3
(22) Date de dépôt: 26.04.1990
(51) Int. Cl.: A61F 5/01

(54) **APPAREIL ORTHOPEDIQUE POUR HANDICAPES D'UNE JAMBE**
ORTHOPÄDISCHE VORRICHTUNG FÜR GEHBEHINDERTE
ORTHOPEDIC APPARATUS FOR PERSONS HANDICAPPED IN ONE LEG

(30) Priorité: 28.04.1989 CH 1623/89; 05.02.1990 CH 351/90
(43) Date de publication de la demande: 17.04.1991
(73) Titulaire: HUMBERT, Charles, CH-2400 Le Locle (CH); JENOURE, Peter, CH-4140 Oberwil (CH)
(72) Inventeur: HUMBERT, Charles, CH-2400 Le Locle (CH); JENOURE, Peter, CH-4140 Oberwil (CH)
(74) Mandataire: Vuille, Roman
(86) Numéro de dépôt international: CH9000113
(87) Numéro de publication internationale: WO9013273

(56) Documents cités:
- WO-A-84/00898
- FR-A- 488 296
- US-A- 1 336 695
- US-A-46 418 82
- Medizinisch-Orthopädische Technik, volume 103, no. 2, mars/avril 1983, (Stuttgart, DE), R. Volkert et al.: "Die Mainzer-Hüftentlastungs-Orthese beim M. Perthes", pages 45-48

## Description

De nombreux dispositifs sont connus pour aider à marcher un homme handicapé d'une jambe. Après la canne et la béquille prise sous l'aisselle, la canne dite "anglaise" s'est beaucoup répandue. Cette canne anglaise comporte un segment parallèle à l'avant-bras, le coude s'appuyant dans une coquille et la main tenant une poignée. A l'usage, des défauts sont apparus, par exemple : la transmission du poids par les bras, le coude et le maintien des cannes par les mains serrées sur les poignées signifie une surcharge de ces organes qui entraîne des fatigues démesurées, voire des inflammations articulaires distales ou proximales, aux membres supérieurs.

La présente invention vise à fournir un appareil orthopédique pour handicapés d'une jambe (phase post-traumatique ou post-opératoire pendant la période nécessitant une décharge totale ou partielle du membre), agencé de façon à éviter les effets secondaires mentionnés et à faciliter la réhabilitation complète.

En vue de ce but, il a été proposé (R. Volkert und Dr. Steeger, 4520 Medizinisch-Orthopädische Technik, Vol. 103 (1983) No 2, Mars-Avril, p. 45-48) un appareil visant à décharger la jambe handicapée et à permettre la marche sans aides des bras ou d'autres moyens orthopédiques. L'appareil proposé enserre la cuisse et soutient la partie dorsale du bassin. Il en résulte que les muscles de la cuisse sont fortement comprimés et que c'est eux qui transmettent le poids du corps à un organe métallique dont l'extrémité inférieure est prévue pour appuyer sur le sol pendant la marche ou en station verticale. Toutefois, le port de cet appareil doit devenir assez rapidement douloureux au niveau de la cuisse.

Il a aussi été proposé (brevet US -A- 4.641.882 lequel représente l'art antérieur le plus proche) dans la même intention un appareil orthopédique comportant un organe d'appui sur le sol, rigide et de forme allongée, muni à son extrémité supérieure d'une selle prévue pour recevoir l'usager assis sur elle comme sur une selle de bicyclette. Cette selle est solidaire de deux poignées que l'usager doit saisir avec ses mains pendant la marche, pour que la selle reste en contact avec son postérieur. La selle ne décharge la jambe handicapée du poids du corps qu'à l'arrêt de l'usager et pas pendant la marche.

La présente invention vise à fournir un appareil orthopédique pour handicapés d'une jambe, qui décharge du poids du corps la jambe handicapée aussi bien à l'arrêt que pendant la marche, sans solliciter les muscles de la cuisse pour transmettre le poids à un organe d'appui sur le sol, et qui laisse les mains et les bras de l'usager entièrement libres.

L'appareil selon l'invention est conforme à la revendication 1.

Les dessins annexés représentent, à titre d'exemples, deux formes d'exécution de l'appareil selon l'invention.
La fig. 1 représente une vue en élévation de profil de la première forme d'exécution;
la fig. 2 montre en perspective cette forme d'exécution portée par un homme;
la fig. 3 montre une vue en élévation de profil de la seconde forme d'exécution;
la fig. 4 est une vue en coupe selon la flèche 4 de fig. 3 de la partie supérieure de la seconde forme d'exécution;
la fig. 5 est une vue de face montrant de quelle façon le poids du corps agit sur la selle de l'appareil.

Les parties correspondantes des différentes exécutions sont désignées par les mêmes numéros de référence.

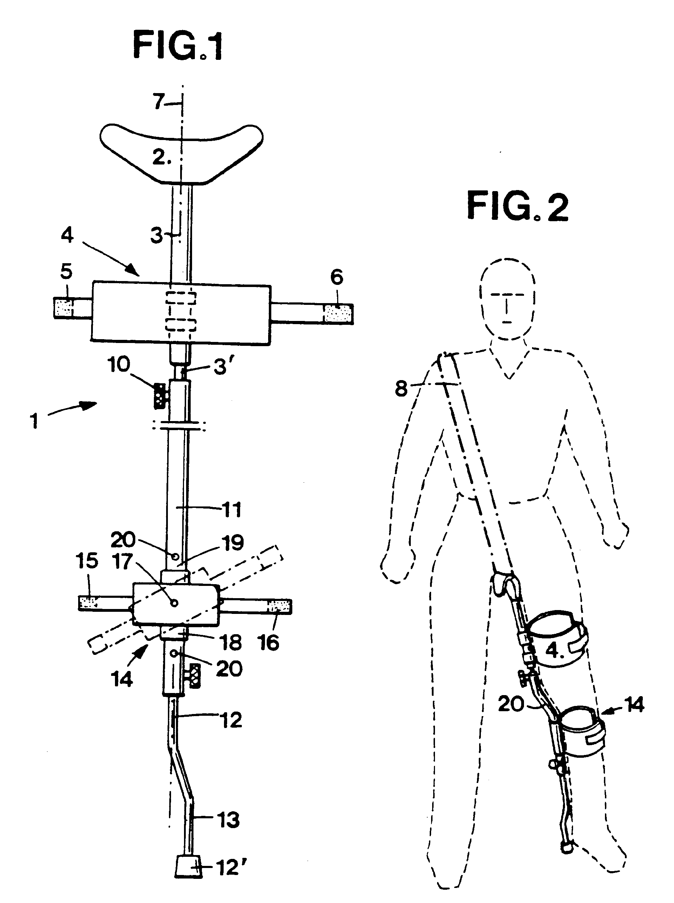

Sur la fig. 1, on a représenté en 1 un organe d'appui sur le sol, qui est rigide, de forme allongée et de longueur réglable. L'organe 1 est muni à sa partie supérieure d'une selle 2 ayant vraiment la forme d'une selle au sens mathématique du terme, c'est-à-dire que dans une coupe axiale le centre de la selle est le point le plus bas et que dans une seconde coupe axiale orthogonale à la première il est le point le plus haut. La selle 2 est fixée solidement à un tube 3 formant la partie supérieure de l'organe d'appui 1 et se prolongeant vers le bas par une tige 3' s'engageant pour glisser dans un tube 11 formant la partie médiane de l'organe d'appui 1. Une vis 10 permet de régler la quantité dont 3' est engagé dans 11. Un dispositif d'attache 4 est solidaire du tube 3; il comporte deux lanières dont les extrémités 5, 6 sont souples et qui, rabattues autour de la jambe, s'accrochent entre elles comme on le voit à la fig. 2. On expliquera plus loin une fonction importante de ce dispositif d'attache.

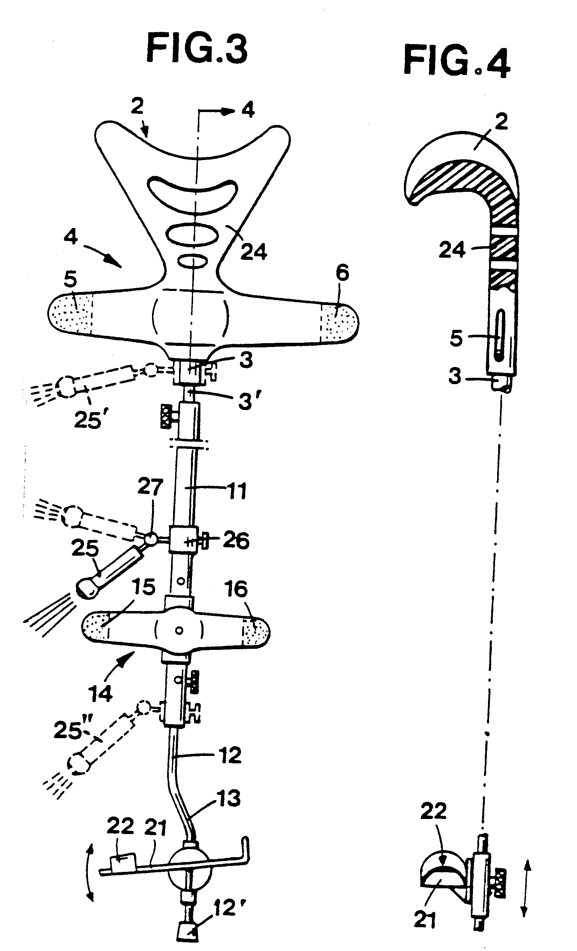

Il est prévu sur le tube 11 un dispositif d'attache 14 comprenant une paire d'attaches latérales 15 et 16 semblables à 5 et 6. Ce dispositif d'attache 14 peut coulisser sur le tube 11 en direction de l'axe longitudinal 7, entre deux butées réglables 20. Il peut aussi tourner d'un certain angle - comme indiqué en traits mixtes à la fig. 1 - autour d'un axe 17 orthogonal à l'axe 7. Un appui plantaire 21, pas représenté dans les figures 1 et 2 mais dans les figures 3 et 4, est prévu pour le soutien du pied de la jambe handicapée.

La partie inférieure de l'organe 1 est formée par un pied tubulaire 12 engagé télescopiquement dans le tube 11 pour former un tout rigide de longueur réglable.

Un plot de caoutchouc 12' assure le contact du pied 12 avec le sol; son élasticité amortit le choc de contact lors de l'emploi de l'appareil pour marcher comme il est expliqué plus loin. Le revêtement de la selle 2 peut également être plus ou moins souple; la pièce 2 elle-même peut présenter une certaine flexibilité.

L'extrémité inférieure du pied 12 est décalée vers l'arrière (comme indiqué en 13) par rapport à l'axe 7 pour jouer le rôle du talon du pied de l'usager (voir fig. 2).

Pour améliorer le maintien du dispositif à sa place, on peut facultativement le compléter par une bretelle 8 passant par dessus l'épaule opposée à la jambe handicapée (fig. 2) et s'accrochant par ses extrémités avant et arrière à la selle 2.

Dans la seconde forme d'exécution (fig. 3 et 4), les dispositifs d'attache sont un peu différents de ceux de la fig. 1. L'appui plantaire 21 est fixé latéralement au tube 12. Avantageusement, cet appui peut osciller légèrement comme indiqué par la flèche de la fig. 3, pour prendre automatiquement la bonne position par rapport au pied qu'il doit supporter; sa place dans la hauteur du pied 13 peut également être réglée, par exemple par un agencement comme indiqué en bas de la fig. 4. Un étrier fixe 22 est placé à l'avant de l'appui plantaire. A chaque pas, lorsque l'usager lève son pied, le dos de son pied soulève, par l'étrier 22, l'appui plantaire 21 et tout le dispositif.

A la place de l'étrier 22 on peut aussi imaginer deux bandes souples, analogues à 5 et 6 de fig. 1, pour serrer le pied et s'accrocher ensemble. Avec l'appui plantaire la jambe handicapée de l'usager est maintenue en trois points 4, 14 et 22; il est possible de lui imposer une position pliée.

Aux fig. 3 et 4, on voit la selle 2 formant un corps 24 avec le premier dispositif d'attaches 4, le tout moulé en plastique rigide, semi-rigide et en partie souple.

Les fixations entre tubes collaborant télescopiquement sont du type de celles utilisées couramment dans ces dispositifs de réhabilitation, par exemple des vis avec des têtes manoeuvrables à la main ou des chevilles encliquetables dans un logement et qui ne peuvent être insérées ou extraites que par une force déterminée.

Dans certains cas il peut être avantageux de proposer à l'usager un dispositif de soutien permettant une modification facile de la longueur totale, manoeuvrable par l'usager. Ainsi, par exemple, pour la marche ou pour la position debout d'attente, des longueurs légèrement différentes sont 'à disposition, commutables par exemple par un levier agissant sur un excentrique.

La forme d'exécution de la fig. 3 est complétée par un éclairage. Une lampe 25 produit un faisceau lumineux dirigé dans le sens de la marche. La lampe 25 est fixée par une bride au tube 11, une articulation 27 se trouvant entre la bride et la lampe pour permettre de régler facilement l'orientation du faisceau lumineux. Selon les cas la lampe 25 peut aussi se trouver dans la position marquée 25' sur la figure 3 - donc attachée au dispositif d'attache 4 - ou la position 25''.

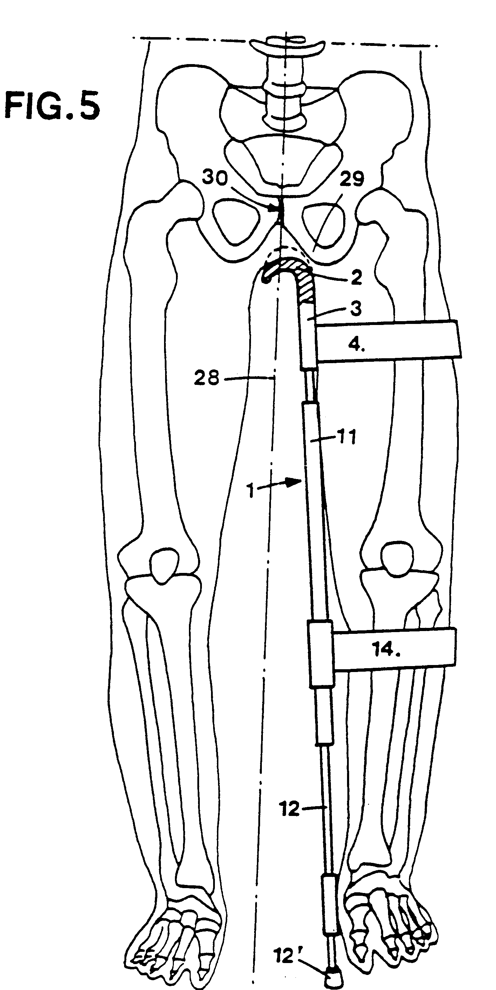

On va expliquer maintenant le fonctionnement des appareils décrits. La partie active de la selle 2 est de largeur notablement inférieure à l'entrejambe de l'usager au niveau de l'aine (voir fig. 4 et 5). Le dispositif d'attache 4 a pour fonction principale d'appliquer la région supérieure (c'est à dire 3 et 11) de l'organe d'appui 1 contre la face intérieure de la cuisse de la jambe handicapée, comme il est visible sur la fig. 5. De ce fait, la surface active (c'est à dire celle prévue pour recevoir le poids du corps) de la selle 2, est maintenue écartée du plan médian 28 du corps de l'usager et en regard de l'os ischion 29 qui est du côté de la jambe handicapée. Ainsi, lorsque 12' s'appuie sur le sol, c'est l'os ischion 29 qui transmet le poids du corps à la selle 2. On évite ainsi de charger la symphyse pubienne 30, qui est une région fragile (surtout chez les femmes enceintes) et pas adaptée à supporter une telle charge.

Le dispositif 4 a une autre fonction importante, qui est d'obliger l'appareil à suivre les mouvements d'oscillation que l'usager donne à sa jambe handicapée, lorsque l'autre jambe supporte momentanément à elle seule le poids total du corps. Ce mouvement d'oscillation demande une force minime et l'usager peut, pour faciliter, donner lors de ces mouvements d'oscillation, un certain écartement du pied vers l'extérieur.

Bien entendu, la présence du second dispositif d'attache 14 facilite le mouvement oscillant de la jambe handicapée avec l'appareil décrit. Toutefois, dans le cas d'un patient dont la jambe handicapée a été amputée, le second dispositif d'attache 14 est inutile et le dispositif d'attache supérieur 4 suffit pour imprimer à la partie supérieure restante de la jambe et à l'appareil un mouvement d'oscillation avant-arrière, pourvu que la partie restante de la jambe mesure au moins 20 cm environ.

Ceci est particulièrement important et avantageux dans le cas de patients venant de subir une amputation et qui ne peuvent pas supporter une prothèse - en raison de la sensibilité du moignon avant une ou même plusieurs années après l'amputation. L'appareil décrit évite ce grave inconvénient et peut être utilisé dès que le patient peut quitter le lit d'hôpital.

On comprend que le fait de pouvoir donner à l'appareil un mouvement pendulaire permet à l'usager de marcher sans utiliser ses bras et des cannes anglaises ou autres et sans employer d'autres moyens orthopédiques. Il peut marcher les mains et les bras complètement libres et sans autre aide que l'appareil décrit. Lors de la marche, le poids du corps est supporté alternativement par la jambe' saine et par l'appareil et la jambe handicapée n'est jamais chargée, même partiellement.

L'usager parvient très rapidement à marcher de façon presque normale et le fait d'avoir les bras libres lui facilite, au début, le maintien de l'équilibre; ensuite il peut même porter des objets tels qu'un parapluie.

Selon la longueur donnée à l'appareil, la plante du pied peut collaborer dans une certaine mesure à la marche, ce qui est très utile pour assurer un certain maintien en fonction du membre handicapé et éviter l'atrophie des muscles de la jambe, ce qui facilite et abrège la réhabilitation.

Lorsque le pied ou le genou de l'usager doit être particulièrement ménagé et/ou que la jambe ne doit pas être maintenue droite, l'appareil permet de fixer sur lui la jambe en trois points: les deux dispositifs d'attache 4 et 14, et l'appui plantaire 21 fixé à la hauteur voulue sur le tube 12, 13.

L'appareil décrit présente encore l'avantage de permettre à l'usager de rester de façon prolongée en station debout, en s'appuyant en grande partie sur l'appareil et dans une mesure moindre sur la jambe saine.

## Revendications

1. Appareil orthopédique pour handicapés d'une jambe, comprenant un organe d'appui sur le sol (1), rigide et de forme allongée, muni à son extrémité supérieure d'une selle (2) prévue pour supporter le poids de l'usager, tandis que son extrémité inférieure (12') est destinée à appuyer sur le sol, caractérisé en ce que la partie active de la selle (2) est notablement plus étroite que l'entrejambe à l'aine de l'usager et est destinée à être engagée entre les jambes de l'usager, et en ce que l'organe d'appui (1) est muni d'au moins un dispositif d'attache (4) à la jambe, pour appliquer la région supérieure (3, 11) de cet organe contre la face intérieure de la cuisse de la jambe handicapée, pour maintenir ainsi la surface utile de la selle (2) écartée du plan médian (28) du corps de l'usager et en regard de l'os ischion (29) de celui-ci qui est du côté de la jambe handicapée, cette attache (4) obligeant l'organe d'appui (1) à suivre les mouvements d'oscillation de la jambe handicapée effectués par l'usager lorsque son autre jambe supporte tout le poids du corps, en ce que la partie inférieure de l'organe d'appui (1) est muni d'un appui plantaire (21) réglable en hauteur et en inclinaison et muni d'une attache (22) formant étrier, pour permettre à l'usager de soulever l'appareil en levant son pied du côté handicapé, et en ce que la partie inférieure (13) de l'organe d'appui (11) est conformée pour s'appuyer sur le sol en arrière du prolongement rectiligne de l'autre partie de cet organe d'appui.

2. Appareil selon la revendication 1, caractérisé en ce qu'il comporte un second dispositif d'attache (14) à la jambe dans la région comprise entre la cheville et le genou et qui est orientable par rapport à l'organe d'appui (1).

3. Appareil selon l'une des revendications 1 à 2, caractérisé en ce qu'il comporte une bretelle dont les extrémités s'accrochent aux extrémités avant et arrière de la selle (2) et qui passe sur l'épaule de l'usager du côté opposé à la jambe handicapée.

4. Appareil selon l'une des revendications 1 à 3, caractérisé en ce qu'il comporte une lampe fixée sur lui de façon réglable en orientation, pour éclairer le sol devant l'usager.

## Claims

1. An orthopedic apparatus for persons handicapped in one leg, comprising a rigid member (1) of an elongated shape for leaning against the ground, provided at its upper end with a saddle (2) for supporting the user's weight, while the lower end (12') is designed for leaning against the ground, characterized in that the active part of the saddle (2) is significantly narrower than the user's crotch at his groin and is designed to be engaged between the user's legs, and in that the leaning member (1) is provided with at least one device (4) for attachment to the leg, for setting the upper region (3, 13) of said member against the inside of the thigh of the handicapped leg, in order to maintain the useful surface of the saddle (2) offset with respect to the medial plane (28) of the user's body and facing the ischium (29) which is on the side of the handicapped leg, said attachment (4) forcing the leaning member (1) to follow the swinging movement of the handicapped leg imparted by the user when his other leg supports the whole weight of the body, in that the lower part of the leaning member (1) is provided with a plantar support (21), the height and the inclination of which can be adjusted and which is provided with an attachment means (22) forming a stirrup enabling the user to lift the apparatus when lifting the leg which is handicapped and in that the lower part (13) of the leaning member (11) is conformed so as to lean against the ground behind the straight line along which the other part of this leaning member extends.

2. An apparatus according to claims 1, characterized in that it includes another attachment device (14) to the leg in the region comprised between the ankle and the knee and which can be oriented with respect to the leaning member (1).

3. An apparatus according to one of claims 1 to 2, characterized in that it comprises a belt, the ends of which are fastened to the front end and to the back end of the saddle (2) and which is passed over the user's shoulder opposite the handicapped leg.

4. An apparatus according to one of claims 1 to 3, characterized in that it comprises a lamp fastened thereto in such a manner as to allow an adjustment of the direction of the beam on the ground in front of the user.

## Patentansprüche

1. Orthopädische Vorrichtung für einseitig Gehbehinderte, bestehend aus einer Bodenstütze (1), die starr und von gestreckter Form ist und an ihrem oberen Ende einen Sattel (2) aufweist, der dazu bestimmt ist, das Gewicht des Benützers abzustützen, während ihr unteres Ende (12') dazu bestimmt ist, auf den Boden aufzusetzen,
dadurch gekennzeichnet, dass der aktive Teil des Sattels (2) merklich schlanker ist als der Schritt an der Leiste des Benutzers und dazu bestimmt ist, zwischen den Beinen des Benutzers gehalten zu werden, und dass die Bodenstütze (1) mit mindestens einer Vorrichtung (4) zur Befestigung am Bein versehen ist, um den oberen Teil (3, 11) der Stütze so an die Innenseite des Oberschenkels des betroffenen Beines zu lehnen, dass auf diese Weise die Nutzfläche des Sattels (2) zur Körpermittelebene (28) des Benutzers seitlich versetzt gegen die Schamleiste (29) auf der Seite des betroffenen Beines gehalten wird, wobei der Befestigungsring (4) die Stütze (1) zwingt, der Schwungbewegungen des betroffenen Beines zu folgen, die der Benutzer ausführt, während das andere Bein das gesamte Körpergewicht trägt, und dass ferner der untere Teil der Stütze (1) mit einer Fussstütze (21) versehen ist, die in ihrer Höhe und Neigung verstellbar ist und einen steigbügelartigen Aufsatz (22) trägt, der es dem Benutzer gestattet, die Stütze mit dem Fuss des geschädigten Beines anzuheben, wobei ein unterer Teil (13) der Stütze (11) so geformt ist, dass er hinter der geradlinigen Fortsetzung des anderen Teils dieser Stütze auf den Boden aufsetzt.

2. Vorrichtung nach Anspruch 1,
dadurch gekennzeichnet, dass sie einen zweiten Befestigungsring (14) aufweist, mit dem sie zwischen Knie und Knöchel am geschädigten Bein befestigt werden kann und der selbst an der Stütze (1) drehbar ist.

3. Vorrichtung nach einem der Ansprüche 1 und 2,
dadurch gekennzeichnet, dass sie einen Tragriemen aufweist, dessen Enden vorn und hinten am Sattel (2) befestigt sind und der über die Schulter auf der dem geschädigten Bein gegenüberliegenden Seite des Benutzers geführt wird.

4. Vorrichtung nach einem der Ansprüche 1 bis 3,
dadurch gekennzeichnet, dass an ihr eine in ihrer Richtung verstellbare Lampe angebracht ist, mit der der Boden in Gehrichtung beleuchtet werden kann.
